# EUROPÄISCHE PATENTANMELDUNG

(11) **EP 2 338 408 A1**
(43) Veröffentlichungstag der Anmeldung: **29.06.2011**
(21) Anmeldenummer: 09016029.2
(22) Anmeldetag: 24.12.2009
(51) Int. Cl.: A61B 5/00, A61M 5/172

(54) **Messende Injektionsvorrichtung**

(71) Anmelder: Roche Diagnostics GmbH, 68305 Mannheim (DE); F.Hoffmann-La Roche AG, 4070 Basel (CH)
(72) Erfinder: Koehler, Matthias, 69514 Laudenbach (DE)
(74) Vertreter: Silber, Anton

(57) **Zusammenfassung**

Die Erfindung betrifft eine messende Injektionsvorrichtung (1), insbesondere zur Injektion einer Insulinlösung und zum gleichzeitigen Messen eines Glucosewertes sowie ein Injektionssystem mit einer eine Messeinrichtung (23) aufweisenden Injektionsvorrichtung (1). Durch geschickte Ausbildung der Injektionsvorrichtung (1) lässt sich die Bedienbarkeit eines Gesamtsystems verbessern.

## Beschreibung

Die vorliegende Erfindung betrifft eine Injektionsvorrichtung, insbesondere zur Injektion einer Insulinlösung und zum gleichzeitigen Messen eines Glucosewertes, und ein Injektionssystem zum Einspritzen einer Injektionsflüssigkeit in den Körper.

Aus der US 6,032,059 ist eine Vorrichtung zur Detektion eines Analytwertes und zur Verabreichung einer therapeutischen Substanz bekannt. Die Vorrichtung ist mit einer Kanüle und mit einem in bzw. an der Kanüle angeordneten Sensor ausgestattet. Dabei detektiert der Sensor den jeweils interessierenden Analytwert in der Körperflüssigkeit. Eine ebenfalls mit dem Sensor verbundene Datenverarbeitungseinrichtung empfängt und verarbeitet die vom Sensor kommenden Signale. Des Weiteren kann das Gerät an eine Dosiervorrichtung angeschlossen werden, die eine Injektionsflüssigkeit über die Kanüle in den Körper injiziert. Eine so kombinierte Dosier- und Messvorrichtung kann mit einem einzigen Einstechen der Kanüle in den Körper einen Analytwert in der Körperflüssigkeit messen, die benötigte Menge an therapeutischer wirksamer Substanz bestimmen und injizieren, ohne dass ein solche Vorrichtung vom Körper entfernt werden muss. Alternativ ist es möglich, während bzw. nach der Injektion der therapeutisch wirksamen Substanz den Analytwert zu messen. In diesem Fall ist die Injektionsmenge vorab festgelegt (z. B. bei einer fixen, vom Arzt vorgegebenen Therapie) und die Bestimmung des Analytwerts dient lediglich der Kontrolle.

In der US 6,758,835 B2 ist eine Einwegkanüle, integriert mit einem Sensor, zur simultanen Vermessung und Verabreichung von Flüssigkeiten über zwei separate Kanülenwege beschrieben. Des Weiteren wurde auch die Ausstattung einer solchen Einwegkanüle mit mehreren Sensoren angedacht. Ebenso kann der Nadelkörper aus einem elastomeren Material aufgebaut sein bzw. ein elastomeres Material enthalten. Auch sind die Öffnungen der beiden Kanülenwege voneinander beabstandet, so dass sich die durch den einen Kanülenweg verabreichte Injektionsflüssigkeit nicht mit der durch den anderen Kanülenweg entnommene Körperflüssigkeit vermischt. Zur Detektion von Analytwerten in der Körperflüssigkeit aber auch zur Erfassung anderer Umgebungsparameter, wie z. B. der Körpertemperatur oder des Blutdruckes können Drucksensoren, optische Sensoren, Temperatursensoren, akustische Sensoren, elektrische Sensoren oder chemische Sensoren verwendet werden.

Das in der WO 2008/038274 A1 beschriebene System zur Flüssigkeitsverabreichung bei gleichzeitiger elektrochemischer Vermessung von Analytkonzentration in der Körperflüssigkeit ist die Kanüle als Rohr-in-Rohr-System ausgebildet. Mit dem inneren Rohr wird die Injektionsflüssigkeit in den Körper injiziert, während das äußere Rohr Aussparungen aufweist, über die die Körperflüssigkeit entnommen werden kann, wobei die Körperflüssigkeit in dem Zwischenraum zwischen dem inneren und dem äußeren Rohr der Kanüle abgeführt wird. Dabei können die Aussparungen im äußeren Rohr semi-permeabel ausgebildet sein, so dass nur die gewünschten Komponenten der Körperflüssigkeit entnommen werden.

Nachteilig an all diesen Systemen, bei denen gleichzeitig eine Injektionsflüssigkeit in den Körper injiziert wird und eine Körperflüssigkeit zu analytischen Zwecken aus dem Körper entnommen wird, ist die aufwendige und kostenintensive Ausgestaltung der Kanüle in Form einer Doppelkanüle oder einer Rohr-in-Rohr-Kanüle.

Die vorliegende Erfindung beschäftigt sich mit dem Problem, für eine Injektionsvorrichtung und für ein Injektionssystem eine verbesserte Ausführungsform anzugeben, die sich insbesondere durch eine kostengünstig herstellbare Kanüle auszeichnet.

Erfindungsgemäß wird dieses Problem durch die Gegenstände der unabhängigen Ansprüche gelöst. Vorteilhafte Ausführungsformen sind Gegenstand der abhängigen Ansprüche.

Die Erfindung beruht auf dem allgemeinen Gedanken, an einer Kanüle einer Injektionsvorrichtung zur Injektion einer Injektionsflüssigkeit in den Körper zumindest eine Messeinrichtung zum Vermessen zumindest eines in einer Körperflüssigkeit auftretenden Analytwertes anzuordnen, wobei die Kanüle an der einer ihrere Oberflächen, vorzugsweise einer Außenoberfläche, eine kapillar wirksame Oberflächenstruktur aufweist, mittels der aufgrund einer Kapillarwirkung der Oberflächenstruktur eine an der Einstichstelle austretende Körperflüssigkeit zur Messeinrichtung transportiert werden kann. Eine solche Kombination einer Kanüle zur Injektion einer Injektionsflüssigkeit in den Körper mit einer Messeinrichtung zum Vermessen zumindest eines in einer Körperflüssigkeit auftretenden Analytwertes hat den Vorteil, dass die Funktion des Vermessens des in einer Körperflüssigkeit auftretenden Analytwertes mit der Funktion der Injektion einer Injektionsflüssigkeit in den Körper zusammengefasst wird. Da bei einem dementsprechenden Erkrankungsbild eine als Injektionsflüssigkeit zu verabreichende therapeutisch wirksame Substanz ohnehin injiziert werden muss, kann durch ein solches Zusammenfassen der Funktionen des Messens und des Injizierens die Anzahl der notwendigen Körperflüssigkeitsentnahmen verringert werden. Dadurch muss der Patient weniger oft gestochen werden, es treten weniger Schmerzen und weniger Hautirritationen auf und ein damit einhergehendes Entzündungsrisiko wird verringert. Des Weiteren ist zudem üblicherweise das Stechen mittels einer Lanzette, z. B. zur Blutentnahme, deutlich schmerzhafter als das Stechen mit einer dementsprechend dünneren Kanüle bei der Verabreichung einer Injektionsflüssigkeit. Zudem wird erlaubt die Erfindung eine Erhöhung der Mess-Compliance, d.h. es werden ausreichend viele Messwerte generiert, so dass der Arzt bei der nächsten Visite die Therapie ggf. optimieren kann. Darüberhinaus ist es nicht erforderlich, den Patienten zusätzlich für eine Analytmessung zu motivieren, denn insbesondere bei fester Therapie, z. B. bei regelmäßiger Basalinjektion mit fester Menge Insulin unabhängig vom aktuellen Blutglukosemesswert, besteht für den Patienten kein direkter Nutzen in einer Blutglukosemessung. Für eine retrospektive Analyse des Blutglikoseverlaufs durch den Arzt jedoch sind regelmäßige Blutglukosemesswerte essentiell zur Einstellung der Therapie.

Bei der Injektion von Flüssigkeiten in den Körper treten als Körperflüssigkeit üblicherweise entweder Blut oder die interstitielle Flüssigkeit aus der Einstichwunde aus. Somit eignet sich eine solche Injektionsvorrichtung zum Vermessen von Analytwerten des Blutes oder der interstitiellen Injektionsflüssigkeit, je nachdem, in welchen Teil des Körpers eine Injektion vorgenommen wird, d. h. ob man gerade ein Blutgefäß trifft (und somit Blut als Probenflüssigkeit vorliegt) oder nicht (und somit die Probenflüssigkeit interstitielle Flüssigkeit (ISF) ist). Falls der Sensor nur entweder für Vollblut oder für ISF kalibiriert werden kann, kann man z. B. grundsätzlich nur für ISF kalibirieren. Für den Fall, dass ausnahmsweise ein Blutgefäß getroffen wird, muss der Sensor erkennen, dass es sich bei der Probenflüssigkeit um Vollblut handelt und die Messung verwerfen. Verfahren zum Erkennen von Vollblut sind aus dem Stand der Technik bekannt. Alternativ ist es denkbar, 1 Messfeld für ISF und parallel 1 Messfeld für Vollblut bereitzustellen.

Dementsprechend können mit einer solchen Injektionsvorrichtung Zucker, bevorzugt Glucose, Lipide, Stoffwechselprodukte, wie z. B. Harnstoff bzw. Harnsäure, Proteine, Peptide und Salze, sowie organische als auch anorganische Phosphate oder andere Inhaltsstoffe des Blutes oder der interstitiellen Flüssigkeit als Analytwert bestimmt werden.

Des Weiteren bietet sich eine solche Injektionsvorrichtung, bei der gleichzeitig zumindest ein in einer Körperflüssigkeit auftretender Analytwert bestimmt wird, bevorzugt bei der Verabreichung einer Injektionsflüssigkeit an, die eine therapeutisch wirksame Substanz enthält, die weder oral noch rektal und auch nicht mittels Resorption über die Haut verabreicht werden kann. Somit ist eine solche Injektionsvorrichtung bevorzugt im Bereich der Diabeteserkrankungen anwendbar, da Insulin bzw. Glucagon Peptidhormone sind, die bei oraler Verabreichung inaktiviert werden und bei rektaler Verabreichung bzw. durch Resorption über die Haut nicht in den Körper aufgenommen werden können. Da im Fall von therapeutisch wirksamen Substanzen, die sinnvoll nur über eine Injektion in den Körper verabreicht werden können, somit eine Injektion mit einer Vermessung der Körperflüssigkeiten kombiniert werden kann, ist eine solche Injektionsvorrichtung unter anderem aufgrund der Schmerzreduktion vorteilhaft.

Es ist der Einsatz einer solchen messenden Injektionsvorrichtung aber auch bei anderen Injektionen, wie z. B. bei der Verabreichung einer isotonischen Elektrolytlösung, bei der Injektion eines Schmerzmittels oder bei dem Einspritzen eines Betäubungsmittels, möglich.

Bevorzugt kann eine solche messende Injektion bei Typ-II-Diabetikern mit einem fixen Verabreichungsplan zum Einsatz kommen. Bei Typ-II-Diabetes mit konventioneller Therapie ist üblicherweise keine Therapieanpassung aufgrund eines vor der Injektion gemessenen Blutglucosewertes notwendig. Diese Typ-II-Diabetiker folgen einem festen Spritzschema, mit z. B. ein bis zwei Injektionen Basalinsulin oder Mischinsulin pro Tag. Da gerade der Typ-II-Diabetiker keinen direkten, unmittelbaren Nutzen aus einer Blutglucosemessung ziehen kann, ist hierfür die Motivation gering, zumal das Blut zur Bestimmung des Blutglucosewertes aus Körperbereichen, wie z. B. aus den Fingerkuppen, entnommen wird, in die ein Einstechen besonders schmerzhaft ist. Demzufolge spritzt er sich viel lieber Insulin, denn das hilft sofort, die Kanülen sind viel dünner und die Injektion wird üblicherweise an weniger schmerzempfindlichen Stellen, wie z.B. der Bauchdecke, durchgeführt. Aufgrund dessen ist bei diesen Patienten die Analytwerterfassung häufig sehr schlecht, da sie nur selten und unregelmäßig die Blutentnahme vornehmen. Deshalb ist bei Typ-II-Diabetikern ein gutes Monitoring wichtig, um die Therapie anpassen und optimieren zu können und dadurch eine Verschlechterung des Gesundheitsbildes mit einhergehenden Sekundärerkrankungen und großen Kosten für das Gesundheitswesen zu vermeiden. Vorteilhafterweise wird bei einer solchen messenden Injektionsvorrichtung parallel zur Injektion auch ein Analytwert, wie z. B. der Glucosewert, der aus der Injektionsöffnung austretenden Körperflüssigkeit vermessen. Dadurch lässt sich gerade bei Typ-II-Diabetikern mit CT das Monitoring der Glucosewerte verbessern und gleichzeitig die Bedienbarkeit vereinfachen, sowie die auftretenden Schmerzen verringern.

Aber auch bei Typ-I-Diabetikern ist der Einsatz einer solchen messenden Injektionsvorrichtung durchaus sinnvoll. Üblicherweise bestimmt der Typ-I-Diabetiker kurz vor der Insulingabe durch Einstechen z.B. in den Finger und Entnahme von Blut aus der Einstichstelle mittels eines Testinstruments den Blutglucose-Wert. Auf diese separate Messung kann entweder verzichtet werden, da auch durch die Injektionsvorrichtung kurz vor oder während der Injektion der Glucosewert in der austretenden Körperflüssigkeit bestimmbar ist oder es kann durch die messende Injektionsvorrichtung der Blutglucosewert der separaten Messung verifiziert werden.

Die Injektionsvorrichtung ist mit einer Kanüle ausgestattet, die eine kapillarwirksame Oberflächenstruktur aufweist. Aufgrund der Kapillarwirkung der Oberflächenstruktur kann die aus der Einstichstelle austretende Körperflüssigkeit zu der an der Kanüle angeordneten Messeinrichtung transportiert werden. Eine solche Oberflächenstruktur kann durch zumindest eine parallel und/oder zumindest teilweise quer zur Längsachse der Kanüle verlaufende Kapillarrinne gebildet werden. Im Falle von parallel zur Längsachse der Kanüle verlaufenden Kapillarrinnen ist eine Anzahl von 1 bis 6 solcher Kapillarrinnen bevorzugt. Es ist aber auch möglich, dass beispielsweise bis zu 10 oder mehr Kapillarrinnen an der Kanüle ausgebildet sind. Vorteilhaft an der Ausbildung solcher Kapillarrinnen ist der gezielte Transport der Körperflüssigkeit von der Einstichstelle zu einem in Umfangsrichtung der Kanüle genau definierten Ort. Dadurch lassen sich an der Kanüle in Umfangsrichtung eine oder mehrere Messeinrichtungen mit dementsprechend zugehörigen Kapillarrinnen ausbilden.

Ebenfalls realisierbar ist ein wendelförmiger Verlauf von ein oder mehreren Kapillarrinnen, vorzugsweise an der Außenoberfläche der Nadel.

Vorteilhaft auf die Kapillarwirkung wirkt sich zudem eine hydrophile Beschichtung und/oder Plasmabehandlung und/oder eine Ausbildung von Nanostrukturen in den Kapillarrinnen aus. Es ist ebenfalls möglich, die gesamte Oberfläche oder zumindest die Außenoberfläche der Nadel und/oder die Kapillarrinne hydrophil zu beschichten und/oder mit Plasma zu behandeln und/oder mit Nanostrukturen zu versehen.

Üblicherweise sollte die in den Körper zu injizierende Injektionsflüssigkeit nicht zu schnell verabreicht werden und die Pennadel ca. 10 sec nach Abschluss der Injektion im Körper verbleiben, um sicher zu stellen, dass das Insulin absorbiert wird und nicht einfach wieder aus dem Körper austritt, so dass die Injektionszeit dazu ausreicht, aufgrund der Kapillarwirkung der Oberflächenstruktur die Körperflüssigkeit von der Einstichstelle zu der Messeinrichtung zu transportieren. Zweckmäßig ist eine Transportzeit für den Transport der Körperflüssigkeit hinsichtlich der Injektionszeit optimiert. Transport kann auch länger dauern, Hauptsache die Nadel ist mit ISF benetzt

Eine solche Messeinrichtung ist mit zumindest einem Testelement ausgestattet, das eine für den jeweiligen Test entwickelte Testchemie enthält. Bevorzugt ist diese Testchemie unempfindlich gegenüber der in den Körper zu injizierenden Injektionsflüssigkeit. Somit wird aufgrund der Unempfindlichkeit der Testchemie gegenüber der zu injizierenden Injektionsflüssigkeit durch eben diese der Test nicht oder nur unwesentlich verfälscht. Allerdings kann durch die zu verabreichende Injektionsflüssigkeit dennoch ein Messfehler auftreten, da durch Einsaugen der zu injizierenden Injektionsflüssigkeit in das Testelement die Körperflüssigkeit nicht oder nur noch teilweise in das Testelement eintreten kann. Aufgrund dessen kann sich in der Messung ein Verdünnungsfehler einschleichen, oder aufgrund des Verdrängungseffektes der zu injizierenden Injektionsflüssigkeit wird eben diese und nicht die Körperflüssigkeit vermessen. Dem könnte Abhilfe geschaffen werden, durch Verwendung eines kapillaraktiven Absaugreservoirs, das in der Abfolge Kanüle-Testelement nach dem Testelement angeordnet ist. Dieses kapillarwirksame Absaugreservoir könnte aufgrund der eigenen, höheren Kapillarwirkung das Testelement nach und nach leer saugen, sodass über die kapillarwirksame Oberflächenstruktur der Kanüle eintretende Körperflüssigkeit auch nach vorhergehender Belegung des Testelements durch die Injektionsflüssigkeit wieder in das Testelement eingebracht werden kann. Zweckmäßig ist in diesem Fall die Testchemie auf eine Durchflussmessung optimiert.

Damit die Körperflüssigkeit aus der kapillaraktiven Oberflächenstruktur in das Testelement übertreten kann, ist das Testelement an der Anbindungsstelle zwischen Testelement und Oberflächenstruktur so auszubilden, dass die Körperflüssigkeit aus der Oberflächenstruktur in das Testelement eingesaugt wird. Dazu ist das Testelement zweckmäßig mit einer kapillaraktiven Komponente, wie z.B. einem Saugvlies oder einem Faserbündel, auszustatten, dessen kapillare Wirksamkeit größer ist, als die Kapillarität der Oberflächenstruktur an der Nadel/auf dem Transportweg. Des Weiteren ist ein Kontakt zwischen der Oberflächenstruktur der Kanüle und der kapillaraktiven Komponente des Testelements sicherzustellen.

Des Weiteren kann die Messeinrichtung einen Sensor aufweisen. Dieser Sensor kann elektrochemisch und/oder optisch ausgebildet sein. Bei einem elektrochemischen Sensor wird aufgrund einer katalytischen Wirkung der Testchemie eine zu bestimmende Substanz der Körperflüssigkeit umgesetzt und diese Umsetzung elektrochemisch vermessen, während bei einem optischen Sensor ein Reaktionsprodukt der Testchemie mit einer in der Körperflüssigkeit enthaltenen Substanz mit Licht einer definierten Wellenlänge bestrahlt wird und der Grad der Absorption dieses Lichtes vermessen wird.

Weiterhin kann die Messeinrichtung mit einem Leitungselement ausgestattet sein. Im Falle eines elektrochemischen Sensors kann dieses Leitungselement eine auf einer Kunststofffolie aufgetragene Metallschicht sein, sowie eine Metallfolie oder ein Metalldraht, der gegebenenfalls durch z.B. ein Spritzgussverfahren in die jeweilige Komponente eingespritzt ist. Auch die Ausbildung des Leitungselements aus leitfähigem Kunststoff ist realisierbar. So könnte z.B. die Kanülenhalterung zumindest teilweise aus einem leitfähigen Kunststoff aufgebaut sein. Im Falle eines optischen Sensors, z.B. eines als Lichtleiter ausgebildeten Leitungselementes oder einer Linsen- und/oder Blenden- und/oder Spiegelanordnung ist das Licht zum Testelement und/oder vom diesem weg übertragbar.

Die Messeinrichtung kann ebenfalls eine elektrische und/oder optische Kontaktstelle aufweisen, über die die Messeinrichtung mit einem peripheren Gerät gekoppelt werden kann. Somit ist die Messeinrichtung über eine elektrische Kontaktstelle mit Strom versorgbar und es können über eine solche elektrische Kontaktstelle Informationen übertragen werden. Über eine optische Kontaktstelle lässt sich Licht zwischen einem peripheren Gerät und der Messeinrichtung übertragen. Im Falle eines optischen Sensors kann eine solche Messeinrichtung sowohl mit einer elektrischen als auch mit einer optischen Kontaktstelle ausgestattet sein.

Bevorzugt ist die Kanüle an einer Kanülenhalterung befestigt, mit der die Kanüle an ein peripheres Gerät, wie z.B. eine Dosiereinrichtung, angekoppelt werden kann. Üblicherweise ist eine solche Kanülenhalterung als eine Schraubkappe oder eine Kappe mit Bajonettverschluss ausgebildet, wobei die Kanüle die Schraubkappe bzw. die Kappe mit Bajonettverschluss durchsetzt. Beim Aufsetzen der mit der Kanüle integrierten Kanülenhalterung kann das zur Dosiereinrichtung orientierte Ende der Kanüle ein Septum des Reservoirs der Injektionsflüssigkeit durchstechen, während üblicherweise eine Spitze der Kanüle zur Injektion in den Körper vorgesehen ist und mit einer abnehmbaren Schutzkappe ausgestattet ist.

Ebenfalls herstellbar ist eine Kanülenhalterung, die nicht wie üblich auf die Dosiervorrichtung aufgeschraubt wird, sondern die eine Magazinierung von mehreren Kanülen analog z. B. zu der Magazinierung von Lanzetten bei Stechhilfen zulässt. Auch in diesem Fall würde die Kanülenhalterung durch die Kanüle durchsetzt sein, sodass das Ende der Kanüle mit dem Reservoir der Injektionsflüssigkeit verbunden werden kann, während die Spitze der Kanüle zur Injektion der Injektionsflüssigkeit in den Körper vorgesehen ist. Eine solche Ausführungsform der Kanülenhalterung ermöglicht eine hohe Sterilität und eine vereinfachte Benutzung. Zudem kann der Benutzer in Folge der Magazinierung davon abgehalten werden, die Sterilität der Kanülenspitze durch Fehlbenutzung zu verschlechtern.

Des Weiteren ist es möglich, die Kanülenhalterung im Falle der Magazinierung so auszubilden, dass sie ebenfalls von der Kanüle durchsetzt wird, aber nicht auf die Dosiervorrichtung aufgeschraubt wird, sondern innerhalb der Dosiervorrichtung durch einen quer zur Längsrichtung der Kanüle orientierten Greifmechanismus aus einem Magazin entnommen wird. Nach Entnahme und Positionierung eines solchen Kanülen-Einweg- und/oder Mehrwegartikels aus Kanüle und Kanülenhalterung kann das Ende der Kanüle mit dem Injektionsflüssigkeitsreservoir verbunden werden, wobei die Spitze der Kanüle gleichzeitig aus der Dosiervorrichtung zur Injektion ausgefahren wird.

Unterschiedliche Komponenten der Injektionsvorrichtung können als Einweg- und/oder Mehrwegartikel ausgebildet sein. So ist es derzeit üblich, die Kanüle mit der Kanülenhalterung als einen solchen Kanülen-Einweg- und/oder Mehrwegartikel auszugestalten. Eine solcher, wie schon vorhergehend beschriebener Kanülen-Einweg- und/oder Mehrwegartikel aus der mit der Kanüle integral ausgebildeten Kanülenhalterung ist derzeit für eine einmalige Verwendung vorgesehen und kann je nach Bedarf auch mehrmals verwendet werden. So ist es denkbar, dass bei mehrmaliger Verwendung der Kanüle das Testfeld nur bei der Erstbenutzung misst und danach verbraucht ist. Alternativ ist es möglich, dass das Messfeld mehrere Segmente aufweist und bei jedem Injektions- /Messvorgang wird jeweils nur eines dieser Segmente verwendet. Z. B. ist es möglich, dass die Flüssigkeit zuerst nur auf das 1. Feld geleitet wird, bei der nachfolgenden 2. Verwendung auf das 2. Feld usw. Dazu kann das mehrere Segmente aufweisende Testfeld mechanisch gedreht werden, so dass bei jeder neuen Verwendung ein unbenutztes Segment zur Verfügung steht. Alternativ kann das Messfeld mehrere hintereinander liegende Segmente aufweisen, von denen zuerst nur das 1. Feld gefüllt wird, beim 2. mal wird nur das 2. gefüllt usw. Es ist denkbar, dies dadurch zu realisieren, dass eine Kapillare vorgesehen ist, von der nacheinander seitlich die Messfelder abgehen, wobei zuerst die Flüssigkeit in das zuerst abgehende Feld transportiert wird, bei der 2. Injektion läuft die Flüssigkeit an dem Abzweig zum 1. Feld vorbei, da das ja bereits gefüllt ist (auch wenn bzw. gerade weil es inzwischen eingetrocknet ist), und in das 2. Feld usw. Vorzugsweise aber ist das Messfeld austauschbar und vor der weiteren Verwendung der Kanüle kann der Benutzer, ggf. unter Zuhilfenahme einer Hilfsvorrichtung, das Messfeld austauschen.

Des Weiteren kann das Testelement mit einem elektrischen und/oder optischen Leitungselement sowie einer Kontaktstelle integral als Einweg- und/oder Mehrwegartikel ausgebildet werden. Ebenso anwendbar ist im Falle eines elektrochemischen Testsystems die integrale Ausbildung des Sensor mit dem Testelement, dem Leitungselement und der Kontaktstelle als Test-Einweg- und/oder Mehrwegartikel. Ein weiterer Test-Einweg- und/oder Mehrwegartikel kann dabei mehrere solche Testelemente aufweisen, die zumindest einen oder auch mehrere Analytwerte aus der Körperflüssigkeit bestimmen können.

Solche Test-Einweg- und/oder Mehrwegartikel können zwischen Kanülenhalterung und Dosiervorrichtung oder zwischen Kanülenspitze und Kanülenhalterung angeordnet sein. Ebenfalls möglich ist eine in der Kanülenhalterung quer zur Längsrichtung der Kanüle angeordnete Öffnung durch die ein solcher Test-Einweg- und/oder Mehrwegartikel eingeschoben werden kann, so dass eine Vermessung der durch die Oberflächenstruktur der Kanüle transportierte Körperflüssigkeit ermöglicht ist. Der große Vorteil dieser Ausführungsform der Erfindung ist darin zu sehen, dass hierfür Standardteststreifen, wie sie in verschiedenen Blutglukosemsssystemen verwendet werden, benutzt werden können. Alternativ können diese Standardstreifen auch ggf. so modifiziert werden, dass sie sowohl für ein herkömmliches Blutzuckermessgerät als auch für das erfindungsgemäße System verwendet werden können.

Bei einer Anordnung eines Test-Einweg- und/oder Mehrwegartikels zwischen der Kanülenhalterung und der Dosiervorrichtung, ist eine getrennte Produktion des Kanülen-Einweg- und/oder Mehrwegartikels und des Test-Einweg- und/oder Mehrwegartikels durchführbar. Nach gesonderter Produktion der beiden Einweg- und/oder Mehrwegartikel ist die Sterilisierung ebenfalls separat durchführbar. Der Zusammenbau der beiden Einweg- und/oder Mehrwegartikel kann im Produktionsprozess stattfinden und zu einer in üblicher Weise einsetzbaren Verpackungseinheit führen. Ebenfalls ein Zusammenbau kurz vor der Benutzung ist durchführbar, wobei im Falle des Zusammenbaus kurz vor der Benutzung eine Applikationshilfe von Vorteil ist, die den Zusammenbau und die Bedienung vereinfacht.

Eine solche Applikationshilfe ist ebenfalls vorteilhaft bei der Anordnung des Test-Einweg- und/oder Mehrwegartikels zwischen Kanülenhalterung und Kanülenspitze. In einem solchen Fall ist die Handhabung der Injektionsvorrichtung um den Zusammenbau mit dem Test-Einweg- und/oder Mehrwegartikel nur unwesentlich gegenüber der gängigen Bedienung erweitert. Derzeit wird der Kanülen-Einweg- und/oder Mehrwegartikel auf die Dosiervorrichtung aufgesetzt und ist damit bereit zur Injektion. In einem weiteren Schritt kann nun mittels einer Applikationshilfe der Test-Einweg- und/oder Mehrwegartikel zwischen Kanülenspitze und Kanülenhalterung positioniert und ggf. mit einem Halterungselement fixiert werden. Somit hat der Benutzer nur noch einen weiteren durch die Applikationshilfe unterstützten Schritt vorzunehmen und hat dann ebenfalls die mit der Dosiervorrichtung gekoppelte messende Injektionsvorrichtung nutzungsbereit.

In einer bevorzugten Ausführungsform ist der Kanülen-Einweg- und/oder Mehrwegartikel mit dem Test-Einweg und/oder Mehrwegartikel so integral ausgebildet, dass hinsichtlich der Bedienbarkeit kein Unterschied zu der Benutzung eines derzeit üblichen Kanülen-Einweg- und/oder Mehrwegartikel besteht. In diesem Fall ist zumindest ein Testelement mit einem Leitungselement, einer Kontaktstelle und ggf. auch einem Sensor integral mit der Kanülenhalterung ausgebildet, die wiederum von der Kanüle durchsetzt wird. Auch eine Anordnung mehrerer Testelemente ist in diesem Fall realisierbar. Ein solcher Einweg- und/oder Mehrwegartikel kann wie gewohnt mit der Dosiereinrichtung verbunden werden und ist ohne einen weiteren Schritt einsetzbar.

Wird das Testelement zwischen der Kanülenhalterung und der Dosiereinrichtung angeordnet, so ist die kapillarwirksame Oberflächenstruktur zumindest teilweise so auszubilden, dass die Kapillarwirkung durch die Kanülenhalterung hindurch bis hin zum Testelement eine aus der Einstichstelle austretenden Körperflüssigkeit transportieren kann. Somit durchsetzt die kapillar wirksame Oberflächenstruktur die Kanülenhalterung.

Im Falle eines optischen Testsystems ist die integrale Ausbildung des Sensors mit einem Einweg- und/oder Mehrwegartikel ggf. zu teuer. In diesem Fall ist es zweckmäßig, das Leitungselement mit zumindest einem Lichtleiter auszugestalten, sodass das Licht zwischen dem Sensor bzw. dem Lichtemitter und dem Testelement übertragen werden kann. Zweckmäßigerweise kann in diesem Fall der Sensor bzw. der Lichtemitter in der Dosiervorrichtung angeordnet werden.

Es ist ebenfalls möglich, zumindest einen Teil der Auswerteelektronik, insbesondere im Falle eines elektrochemischen Testsystems, direkt in dem Test-Einweg- und/oder Mehrwegartikel unterzubringen. Dabei lassen sich Kalibrierdaten, Chargeninformationen oder dergleichen in der Auswerteelektronik berücksichtigen, so dass anstatt des Messwertes direkt der Analytwert übertragen werden kann. Dadurch ist ein solcher Test-Einweg- und/oder Mehrwegartikel weniger abhängig von einer spezifischen Auswerteelektronik.

In einer bevorzugten Ausführungsform kann eine Dosiereinrichtung, die mit der messenden Injektionsvorrichtung koppelbar ist, eine Datenverarbeitungsvorrichtung, eine Eingabeeinrichtung, eine Ausgabeeinrichtung, einen Datenspeicher, eine Schnittstelleneinrichtung und ggf. einen elektrischen Energiespeicher unter zusätzlicher Verwendung von Energiewandlern aufweisen.

Mittels der Eingabeeinrichtung in Form von Tasten oder Bedienfeldern kann der Benutzer bestimmte Eingaben an der Dosiereinrichtung vornehmen. Eine Ausgabeeinrichtung kann z. B. ein Display umfassen, auf dem zumindest ein gemessener Analytwert anzeigbar ist. Des Weiteren kann die Ausgabeeinrichtung eine optische, haptische oder akustische Signalausgabe aufweisen, wobei je nach Ausgabesignal z. B. das Ende der Messung oder eine fehlerhafte Messung angezeigt werden können.

Eine Datenverarbeitungsvorrichtung, die in der Dosiereinrichtung integriert ist, kann die von dem Testelement und dem Sensor detektierten Werte in die tatsächlichen Analytwerte umrechnen, wobei ggf. Chargendaten, Kalibrierdaten oder dergleichen zu berücksichtigen sind. Weiterhin ist über die Datenverarbeitungsvorrichtung eine Abspeicherung dieser Informationen in dem Datenspeicher durchführbar, der über die Schnittstelleneinrichtung auslesbar ist. Auch ist es vorteilhaft, eine Steuerungssoftware in dem Datenspeicher abzulegen, die für den ordnungsgemäßen Betrieb der Datenverarbeitungsvorrichtung sorgt.

Die Schnittstelleneinrichtung kann kabelgebunden oder kabelfrei ausgestaltet sein, wobei sich derzeit als kabelfreie Schnittstelle eine Technik wie z. B. Bluetooth, WLAN (Wireless Local Area Network), GSM (Global System for Mobile Communication), UMTS (Universal Mobile Telecommunications System), DECT (Digital Enhanced Cordless Telecomunication), Infrarot, Near Field Communication (NFC) und/oder RFID (Radio Frequency Identification) anbieten. Eine kabelgebundene Schnittstelle kann z. B. über eine derzeit übliche Technik wie z. B. USB (universal serial bus), Firewire und/oder eine serielle oder parallele Schnittstellentechnik wie z.B. RS232 realisiert werden.

Zur Energieversorgung kann die Dosiereinrichtung ebenfalls mit einem elektrischen Energiespeicher, wie z.B. einer Batterie, einem Akkumulator, einem Kondensator oder einem Doppelschichtkondensator ausgestattet werden. Es ist hierbei allerdings auch möglich, den Energiespeicher mit Energiewandlern zu koppeln, um die Aufladezyklen für den elektrischen Energiespeicher zu verlängern. Ggf. kann der elektrische Energiespeicher auch über eine drahtgebundene Schnittstelleneinrichtung mit Energie versorgt werden, wie derzeit bei der USB-Technik üblich ist. Es ist auch möglich, die Energie, die beim Eindrücken des Injektionsknopfes entsteht, über einen Piezo in elektrische Energie zu wandeln und so für die Messung zu verwenden.

Werden vorzugsweise mehrere Testelemente verwendet, so können z. B. mehrere Analytwerte bestimmt werden. Mit mehreren Testelementen ist es aber auch möglich, nur einen Analytwert zu bestimmen und aufgrund der Mehrfachmessung das Messergebnis zu verifizieren, um eine höhere Messwertsicherheit sicherzustellen. Insbesondere bei der Messung der Blutglucose aus interstitiellen Injektionsflüssigkeit wird die Messung verfälscht, wenn an der Injektionsstelle anstatt oder zusätzlich zu der interstitiellen Injektionsflüssigkeit Blut austritt, da aufgrund der Injektion ein Blutgefäß verletzt worden ist. In diesem Fall ist es vorteilhaft das Testelement so auszugestalten, dass eine Vollbluterkennung durch dieses Testelement durchgeführt wird. Ein Messfehler ist ebenfalls vermeidbar, wenn mit der austretenden Körperflüssigkeit sowohl ein auf interstitielle Injektionsflüssigkeit optimierter Test durchgeführt wird und durch ein anderes Testelement ein auf Vollblut optimierter Test. Durch Vergleich der ermittelten Werte mit jeweils zu erwartenden Referenzbereichen, kann bestimmt werden, welcher Messwert verworfen werden sollte.

Ist die Dosiereinrichtung mit einer Datenverarbeitungseinrichtung ausgestattet, so kann von der Dosiervorrichtung aufgrund z.B. der Tageszeit, dem Benutzer der Dosiervorrichtung ein Vorschlagswert für die zu injizierende Menge unterbreitet werden. Diesen Vorschlagswert kann der Benutzer vorab an der Dosiereinrichtung einstellen und sich dann zur Injektion die Kanüle in den Körper einführen. Nun wäre es möglich, dass der Benutzer so lange mit der Injektion wartet oder die Injektion solange in die Länge zieht, bis die Injektionsvorrichtung aufgrund der an der Einstichstelle austretenden Körperflüssigkeit, die dementsprechenden Analytwerte ermittelt hat. Aufgrund dieser ermittelten Analytwerte ist durch die Datenverarbeitungsvorrichtung dem Benutzer eine Korrektureinstellung bzgl. der Injektionsflüssigkeit mitteilbar. Diese Korrektureinstellung kann der Benutzer vornehmen, um die korrigierte Menge an Injektionsflüssigkeit zu injizieren. Dieser Vorgang ist auch automatisiert durchführbar, in dem während der Injektion aufgrund der Vermessung der Körperflüssigkeit, die zu injizierende Flüssigkeitsmenge durch die Dosiereinrichtung selbst angepasst wird.

Sämtliche Daten, die von der Dosiervorrichtung über die Datenverarbeitungseinrichtung erhoben werden, können mittels der Schnittstelleneinrichtung an ein peripheres Gerät, wie z. B. einen PC, übertragen werden. Aufgrund dieser Informationen, wie z. B. einer Injektionsmenge, eines Injektionszeitpunkt, der vermessenen Analytwerte der Körperflüssigkeit, kann ein Therapieplan auch unter Berücksichtigung von historischen Daten ggf. durch den behandelnden Arzt oder durch den Benutzer selbst optimiert werden. Da in diesem Fall eine Fülle von Informationen durch die Dosierhilfe und somit nur durch ein medizinisches Instrument erhoben wird, ist die Zuverlässigkeit und Vollständigkeit der Datenreihen erheblich verbessert.

Es ist sogar realisierbar, auf ein Blutzuckermessinstrument vollständig zu verzichten und nur die Dosiervorrichtung im Zusammenspiel mit der messenden Injektionsvorrichtung als einziges Mess- und Verabreichungsinstrument zu verwenden.

Weitere wichtige Merkmale und Vorteile der Erfindung ergeben sich aus den Unteransprüchen, aus den Zeichnungen und aus der zugehörigen Figurenbeschreibung anhand der Zeichnungen.

Es versteht sich, dass die vorstehend genannten und die nachstehend noch zu erläuternden Merkmale nicht nur in der jeweiligen angegebenen Kombination, sondern auch in anderen Kombinationen oder in Alleinstellung verwendbar sind, ohne den Rahmen der vorliegenden Erfindung zu verlassen.

Bevorzugte Ausführungsformen der Erfindung sind in den Zeichnungen dargestellt und werden in der nachfolgenden Beschreibung näher erläutert, wobei sich gleiche Bezugszeichen auf gleiche oder ähnliche oder funktional gleiche Bauteile beziehen.

Es zeigen jeweils schematisch:
- Figur 1:: einen Längsschnitt durch eine Kanüle im Bereich einer Anbindung an eine Dosiervorrichtung,
- Figur 2:: aufgeteilt auf vier Teilfiguren a) bis d) die Kanüle mit zumindest einer jeweils unterschiedlich angeordneten und/oder ausgebildeten Kapillarrinne, insbesondere Teilfigur:
a) eine an einer Kanülenspitze beginnende längs zu einer Längsrichtung der Kanüle verlaufende Kapillarrinne,
b) eine von der Kanülenspitze beabstandet beginnende und in der Längsrichtung der Kanüle verlaufende Kapillarrinne,
c) eine in der Längsrichtung der Kanüle verlaufende und von einer Kanülenöffnung beabstandet beginnende Kapillarrine,
d) eine wendelförmig verlaufende Kapillarrine,

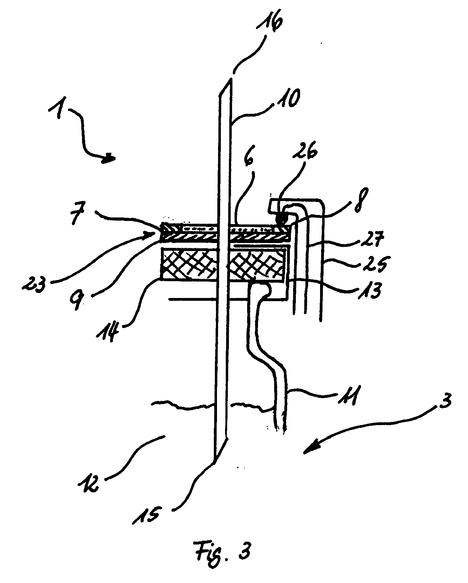

- Figur 3:: eine als Einweg- und/oder Mehrwegartikel ausgebildete Kanüle mit zumindest einem Testelement und einer Kontaktstelle,

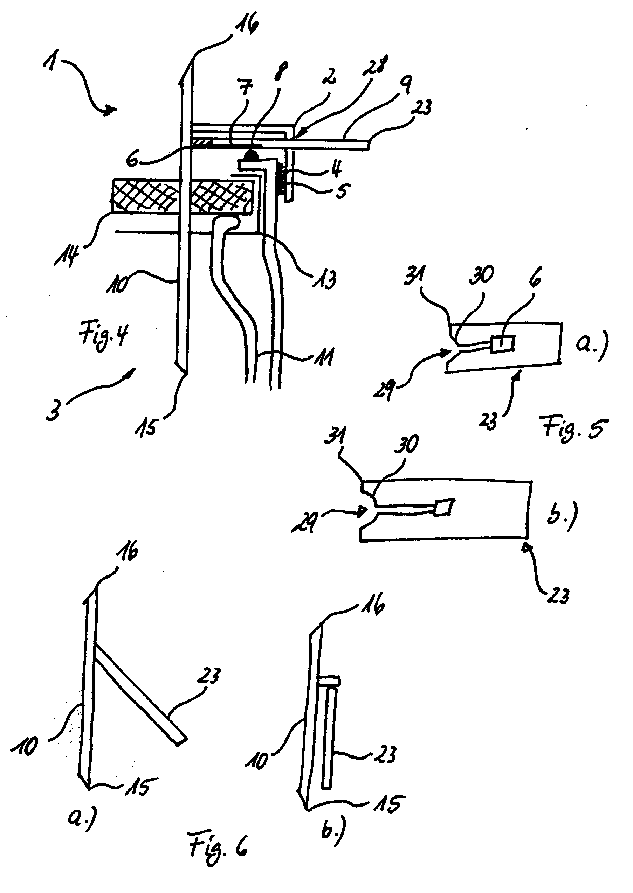

- Figur 4:: einen Kanülen-Einweg und/oder Mehrwegartikel mit einem durch eine Öffnung der Kanülenhalterung eingesteckten Test-Einweg- und/oder Mehrwegartikel,
- Figur 5:: aufgeteilt auf zwei Teilfiguren a) und b) unterschiedliche bevorzugte Ausführungsformen eines einsteckbaren Test-Einweg- und/oder Mehrwegartikels gemäß Figur 4, insbesondere Teilfigur
a) einen Test-Einweg- und/oder Mehrwegartikels mit einer trapezförmigen Aussparung,
b) einen Test-Einweg- und/oder Mehrwegartikels mit einer kreisförmigen oder elipsoiden Aussparung,
- Figur 6:: aufgeteilt auf zwei Teilfiguren a) und b) mögliche Anordnungen des Test-Einweg- und/oder Mehrwegartikels relativ zur Kanüle,
a) eine schräge Anordnung des Test-Einweg- und/oder Mehrwegartikels relativ zur Kanüle,
b) eine parallele Anordnung des Test-Einweg- und/oder Mehrwegartikels relativ zur Kanüle,

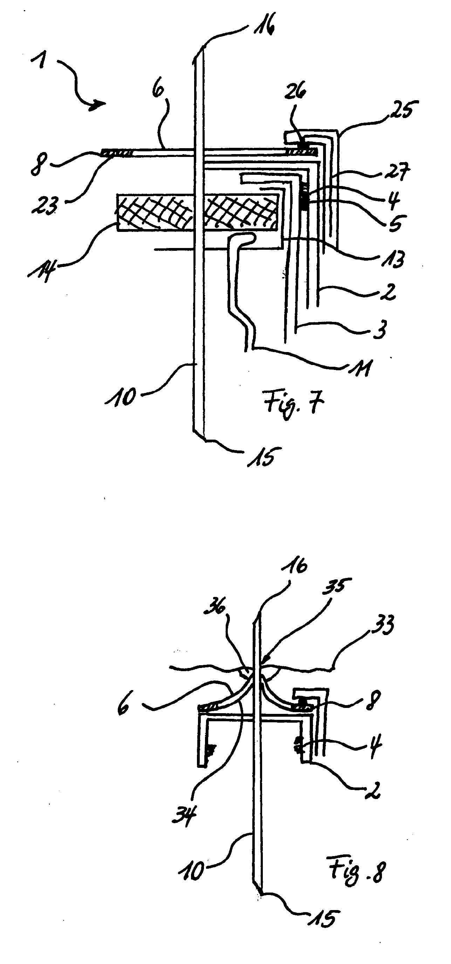

- Figur 7:: eine Injektionsvorrichtung mit zwischen Kanülenhalterung und Kanülenspitze angeordnetem Test-Einweg- und/oder Mehrwegartikel, der durch eine zusätzliche Halterung fixiert wird,
- Figur 8:: einen zwischen Kanülenspitze und Kanülenhalterung angeordneten Test-Einweg- und/oder Mehrwegartikel mit Auswölbung.

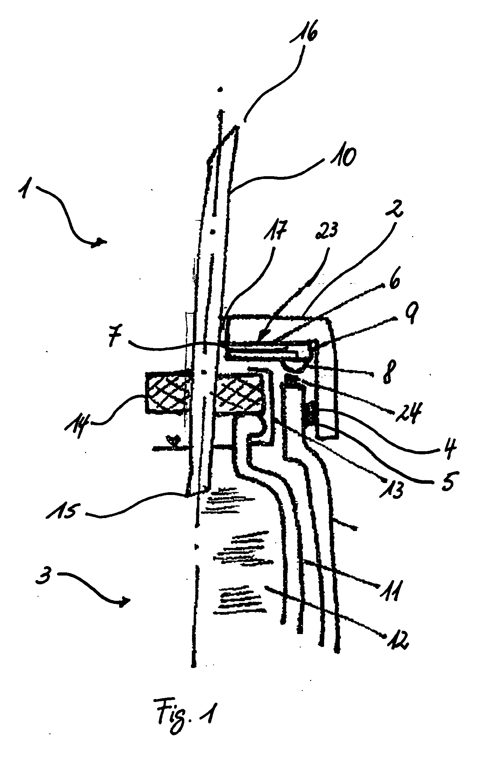

Wie in Figur 1 dargestellt, ist die Injektionsvorrichtung 1 mittels der Kanülenhalterung 2 an die Dosiervorrichtung 3 angekoppelt. Diese Kopplung der Kanülenhalterung 2 an die Dosiervorrichtung 3 wird durch ein Verschrauben des Innengewindes 4 der Kanülenhalterung auf das Außengewinde 5 der Dosiervorrichtung vorgenommen. Aber auch andere Kopplungsmechanismen, wie z. B. ein Bajonettverschluss anstatt eines Schraubverschlusses, sind in anderen Ausführungsformen anwendbar.

Des Weiteren weist die Injektionsvorrichtung 1 ein Testelement 6, ein Leitungselement 7 und eine Kontaktstelle 8 auf. Im Falle eines elektrochemischen Testsystems ist der elektrochemische Sensor integral mit dem Testelement 6 und dem Leitungselement 7 ausgebildet. Zweckmäßig kann das Leitungselement 7 und das Testelement 6 zumindest teilweise von einer Hülle 9 aus z. B. Kunststoff oder dergleichen umgeben sein.

Vorteilhaft können eine Kanüle 10 sowie die Kanülenhalterung 2 als ein Kanülen-Einweg- und/oder Mehrwegartikel ausgebildet sein. Ebenso ist es möglich, dass das Testelement 6 das Leitungselement 7 und die Kontaktstelle 8 als Test-Einweg- und/oder Mehrwegartikel ausgebildet sind. Eine Ausbildung der Injektionsvorrichtung aus mehreren Einweg- und/oder Mehrwegartikeln hat bezüglich der Bedienbarkeit für den Benutzer eine erhöhte Flexibilität. Allerdings kann es auch hinsichtlich der Messwerteerfassung gewünscht sein, dass die Kanüle 10, die Kanülenhalterung 2 sowie das Testelement 6, das Leitungselement 7 und die Kontaktstelle 8 als ein integrierter Einweg- und/oder Mehrwegartikel ausgebildet sind. In diesem Fall wird bei jeder Injektion auch automatisch zumindest ein Analytwert in der aus der Einstichstelle austretenden Körperflüssigkeit bestimmt.

Die Dosiervorrichtung 3 umfasst im Wesentlichen eine Kartusche 11, die eine Injektionsflüssigkeit 12 enthält, wobei die Kartusche 11 durch ein mittels einer Verplombung 13 fixiertes Septum 14 verschlossen ist. Bei der Kopplung der Injektionsvorrichtung 1 mit der Dosiervorrichtung 3 wird das Septum 14 durch ein der Dosiervorrichtung zugewandtes Kanülenende 15 der Kanüle 10 durchstochen, sodass die Injektionsflüssigkeit 12 aus einer Kanülenspitze 16 austreten kann. Mittels weiterer nicht dargestellter Komponenten der Dosiervorrichtung kann dabei die aus der Kanülenspitze 16 austretende Menge an Injektionsflüssigkeit 12 dosiert werden.

Damit die aus der Einstichstelle austretende Körperflüssigkeit zu dem Testelement 6 gelangen kann, ist es vorteilhaft, in der Kanülenhalterung 2 bei einer Anordnung des Testelementes 6 zwischen Kanülenhalterung und Dosiervorrichtung 3, wie in Fig. 1 gezeigt, eine Aussparung 17 vorzusehen. Durch die Aussparung 17 kann die Körperflüssigkeit zu dem Testelement geleitet werden. Zu diesem Zweck ist an der Kanüle 10 zumindest eine Oberflächenstruktur in Form z. B. einer Kapillarrinne 18 ausgebildet, wobei die Oberflächenstruktur kapillar wirksam ist und aufgrund der Kapillarwirkung der Oberflächenstruktur die Körperflüssigkeit zu dem Testelement 6 transportiert.

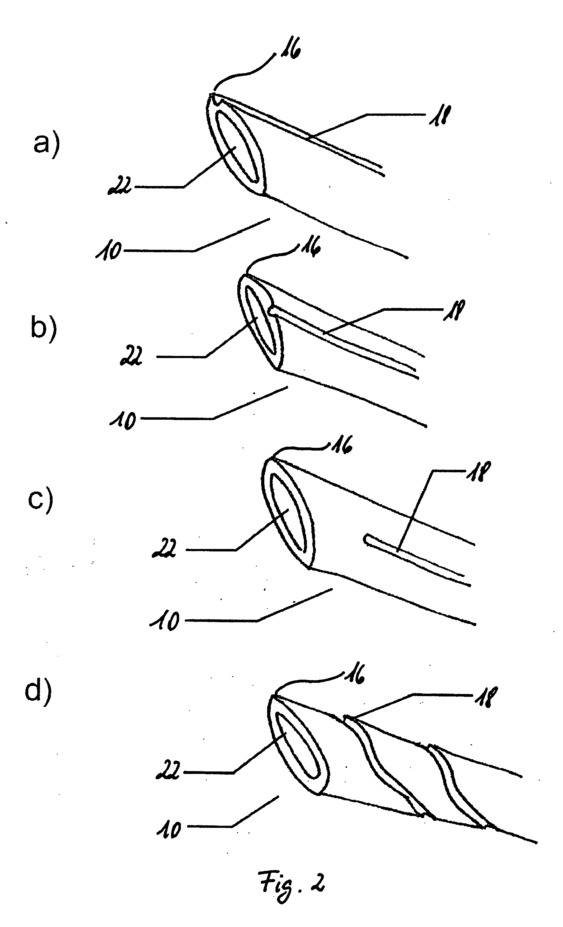

In einer bevorzugten Ausführungsform gemäß Figur 2 ist die kapillarwirksame Oberflächenstruktur als Kapillarrinne 18 ausgebildet. Dabei kann die Kapillarrinne 18 parallel zur Längsachse der Kanüle 10, wie in den Figuren 2a) bis 2c) gezeigt, verlaufen oder gemäß der Figur 2d) einen wendelförmigen Verlauf aufweisen. Des Weiteren kann die Kapillarrinne 18 an einem äußersten Ende der Kanülenspitze 16 beginnen, wie in Figur 2a) dargestellt oder an einer von dem äußersten Ende beabstandeten Stelle der Kanülenspitze 16. Ist ein Beginn der Kapillarrinne 18 einen Abstand weit von der Kanülenspitze 16 entfernt, so ist die Gefahr eines Eindringens der Injektionsflüssigkeit 12 in die Kapillarrinne 18 verringert. Des Weiteren weist die Kanüle 10 an der Kanülenspitze 16 eine Öffnung 22 auf, durch die die Injektionsflüssigkeit 12 austreten kann.

Eine Messeinrichtung 23 der Injektionsvorrichtung 1 umfasst zumindest das eine Testchemie enthaltenes Testelement 6. Dabei ist es realisierbar, dass die Kanüle 10 mit dem Testelement 6 und ggf. der Kanülenhalterung 2 integral ausgebildet ist. Eine solche Ausbildung bietet sich im Falle eines optischen Testsystems an, da in diesem Fall der optische Sensor und/oder der Lichtemitter an der Dosiervorrichtung angebunden sein kann. In diesem Ausführungsbeispiel kann dann sowohl auf ein Leitungselement als auch auf eine elektrische Kontaktstelle verzichtet werden.

Des Weiteren kann die Messeinrichtung auch mehrere Testelemente 6 enthalten, die jeweils einen anderen Analytwert der Körperflüssigkeit bestimmen. Ebenso ist eine Bestimmung des gleichen Analytwertes durch mehrere Testelemente 6 durchführbar, wobei über die Messwerte hinweg gemittelt werden kann, um eine höhere Messwertsicherheit zu erreichen oder wobei unterschiedliche Messmethoden zu einem gleichen Analytwert unter Berücksichtigung der jeweils austretenden Körperflüssigkeit durchgeführt werden können.

Eine solche Messeinrichtung kann ebenfalls einen Sensor zur Erfassung des Analytwertes und/oder ein Leitungselement 7 und/oder eine elektrische und/oder optische Kontaktstelle 8 unter anderem zum Datenaustausch aufweisen.

In einer anderen Ausführungsform gemäß Figur 3 ist die Kanüle 10 integral mit der Messeinrichtung 23 ausgebildet ist. Dabei umfasst die Messeinrichtung 23 ein Testelement 6, eine Kontaktstelle 8 und ein Leitungselement 7, wobei diese Komponenten 6, 7, 8 noch von einer stabilisierenden Hülle 9 zumindest teilweise umgeben sind. Ein solcher integrierter Einweg- und/oder Mehrwegartikel kann z.B. mittels einer Applikationshilfe durch das Septum 14 mit seinem Kapillarende 15 in die Kartusche 11 mit der Injektionsflüssigkeit 12 eingeführt werden. Durch eine separate Halterung 25, die z. B. mit der Dosiervorrichtung 3 verbindbar ist oder integral mit dieser ausgebildet ist, lässt sich ein solcher Einweg- und/oder Mehrwegartikel so an der Dosiervorrichtung 3 fixieren, dass eine Injektion und/oder Messung problemlos durch den Benutzer durchgeführt werden kann. Mittels einer Kontaktstelle 26 und über ein Leitungselement 27 ist eine Weitergabe der durch die Messeinrichtung 23 bestimmten Analytwerte an einen Datenspeicher oder eine Datenverarbeitungseinrichtung der Dosiervorrichtung 3 möglich.

In einer weiteren Ausführungsform im Falle eines optischen Messsystems kann auf die Kontaktstelle 8 sowie das Leitungselement 7 der Messeinrichtung 23 verzichtet werden. In diesem Fall kann allerdings entweder die Kontaktstelle 26 als optische Kontaktstelle ausgebildet sein, mit der optische Signale zwischen der optischen Kontaktstelle 26 über das als Lichtleiter ausgebildete Leitungselement 27 übertragen werden oder die Kontaktstelle 26 kann als integrierter Sensor bzw. Lichtemitter ausgebildet sein, um das Absorptionsverhalten des Testelementes 6 zu vermessen.

In einer anderen Ausführungsform, wie in Figur 4 dargestellt, ist die Messeinrichtung 23 als ein Test-Einweg- und/oder Mehrwegartikel ausgebildet, der derart seitlich durch eine Öffnung 28 der Kanülenhalterung 2 in dieselbe einschiebbar ist, dass das Testelement 6 mit der Kanüle 10 in Kontakt kommt. Diese Ausführungsform hat den Vorteil, dass flexibel, je nach Bedarf, entschieden werden kann, ob während der Injektion ein Analytwert aus der Körperflüssigkeit bestimmt werden soll oder nicht. Ebenso ist es hierbei möglich durch Einschieben mehrerer Messeinrichtungen 23 nacheinander mehrere Analytwerte aus der Körperflüssigkeit zu bestimmen. In einem solchen Ausführungsbeispiel kann die Kanülenhalterung 2 in Form einer Schraubkappe mit einem Innengewinde 4 oder in Form einer Verbindungskappe mit einem Bajonettverschluss ausgebildet sein.

Mögliche Ausführungsformen von als Test-Einweg- und/oder Mehrwegartikel ausgebildeten Messeinrichtungen 23, wie in Figur 5a) und 5b) dargestellt, können mit einer Einführhilfe 29 in Form einer Aussparung 30, die an einem zur Kanüle 10 zugewandten Ende 31 der Messeinrichtung 23 angeordnet ist, und einem zwischen der Aussparung 30 und dem Testelement 6 angeordneten Führungsschlitz ausgestattet sein. Wie in Figur 5a) dargestellt, weist die Aussparung 30 eine trapezförmige Form auf oder ist gemäß Figur 5b) in Form eines Kreis- bzw. Elipsenausschnittes ausgebildet.

Abweichend zu der in Figur 4 dargestellten senkrecht zur Kanüle 10 ausgebildeten Anordnung der Messeinrichtung 23 ist auch eine zur Kanüle 10 schräg orientiertes Einschieben der Messeinrichtung 23 gemäß Figur 6a) bzw. parallel zur Längsrichtung der Kanüle 10 ausgebildete Anordnung der Messeinrichtung 23, wie in Figur 6b) gezeigt, möglich.

In einer weiteren, in Figur 7 gezeigten Ausführungsform ist die Kanüle 10 mit der Kanülenhalterung 2 als Kanülen-Einweg- und/oder Mehrwegartikel ausgebildet. Üblicherweise ist in dieser Ausführungsform die Kanülenhalterung 2 als eine mit der Dosiervorrichtung über ein Innengewinde 4 und Außengewinde 5 ausgebildete Schraubverbindung ausgestaltet. In dieser Ausführungsform wird die Messeinrichtung 23, umfassend zumindest eine solche Kontaktstelle 8 und ein solches Testelement 6, sowie ggf. das Leitungselement 7 und die zumindest teilweise die Komponenten 6, 7, 8 umschließende Hülle (9), zwischen der Kanülenspitze 16 und der Kanülenhalterung 2 angeordnet. Zur Fixierung der Messeinrichtung 23 ist die Halterung 25 vorgesehen, die über die Kontaktstelle 26 und das Leitungselement 27 eine Datenübermittlung zwischen der Kontaktstelle 8 und dem Datenspeicher bzw. der Datenverarbeitungsvorrichtung der Dosiervorrichtung 3 sicherstellt. Eine Verwendung der Messeinrichtung 23 kann mittels einer Applikationshilfe vereinfacht werden. Dabei wird nach dem Aufschrauben der mit der Kanülenhalterung 2 integral ausgebildeten Kanüle 10 auf die Dosiervorrichtung 3 in die Messeinrichtung 23, ggf. durch die Applikationshilfe unterstützt, eingestochen, wobei nach dem Zurückziehen der mit der Dosiereinrichtung 3 verbundenen Injektionsvorrichtung 1 zumindest eine Messeinrichtung 23 aus einem Magazin entnommen wird. Diese Messeinrichtung 23 wird durch die Halterung 25 an der Injektionsvorrichtung 1 sowohl fixiert, als auch über die Kontaktstelle 8 leitend verbunden. An einer solchen Anordnung der Messeinrichtung 23 ist besonders vorteilhaft, dass die kapillarwirksame Oberflächenstruktur nicht durch die Kanülenhalterung 2 hindurchgeleitet werden muss, da die Messeinrichtung 23 vor der Kanülenhalterung 2 angeordnet ist. Des Weiteren ist es in diesem Fall möglich die integral mit der Kanülenhalterung 2 ausgebildete Kanüle 10 als Mehrwegartikel auszubilden, während die Messeinrichtung 23 als Einwegartikel ausgebildet sein kann.

Falls Leitungselemente 7 in Form eines elektrischen Leitungselements oder eines Lichtleiters in bzw. auf der Oberfläche der Kanülenhalterung 2 bzw. der Halterung 25 vorgesehen sind, lässt sich eine solche Anordnung von Leitungselementen 7 sehr einfach durch Aufspritzen bzw. Einspritzen des jeweiligen Leitungselementes 7 in die jeweilige Halterung 2, 25 realisieren. Insofern sind auch Einweg- bzw. Mehrwegartikel mit Leitungselementen 7 kostengünstig darzustellen.

Im Falle der Anordnung der Messeinrichtung 23 zwischen der Kanülenhalterung 2 und der Kanülenspitze 16, wie in Figur 7 dargestellt, kann durch das Anordnen der Messeinrichtung 23 zwischen Kanülenhalterung 2 und Kanülenspitze 16 durch das Einstechen in die Messenrichtung 23 während der Entnahme aus z. B. der Applikationshilfe eine zur Hautoberfläche 33 hin orientierte Auswölbung 34 der Messeinrichtung 23 auftreten. Da an der Einstichstelle 35 eine Körperflüssigkeit 36 austritt und somit die Auswölbung 34 mit dieser Körperflüssigkeit 36 in Kontakt kommen kann, ist es realisierbar, dass sogar ohne eine kapillar wirksame Oberflächenstruktur an der Kanüle 10 aufgrund der Kapillarwirkung des Testelementes 6 selbst die Körperflüssigkeit in das Testelement 6 gesaugt wird und somit eine Vermessung zumindest eines Analytwertes der Körperflüssigkeit 36 ermöglich ist.

### Bezugszeichenliste

- 1: Injektionsvorrichtung
- 2: Kanülenhalterung
- 3: Dosiervorrichtung
- 4: Innengewinde
- 5: Außengewinde
- 6: Testelement
- 7: Leitungselement
- 8: Kontaktstelle
- 9: Hülle
- 10: Kanüle
- 11 1: Kartusche
- 12: Injektionsflüssigkeit
- 13: Verplombung
- 14: Septum
- 15: Kanülenende
- 16: Kanülenspitze
- 17: Aussparung
- 18: Kapillarrine
- 22: Öffnung
- 23: Messeinrichtung
- 24: Kontaktstelle
- 25: Halterung
- 26: Kontaktstelle
- 27: Leitungselement
- 28: Öffnung
- 29: Einführhilfe
- 30: Aussparung
- 31 1: Ende
- 33: Hautoberfläche
- 34: Auswölbung
- 35: Einstichstelle
- 36: Körperflüssigkeit

## Patentansprüche

1. Injektionsvorrichtung, insbesondere zur Injektion einer Insulinlösung und zum gleichzeitigen Messen eines Glucosewertes, mit einer Kanüle (10) zur Injektion einer Flüssigkeit in den Körper, mit zumindest einer an der Kanüle (10) angeordneten Messeinrichtung (23) zum Vermessen zumindest eines in einer Körperflüssigkeit auftretenden Analytwertes, wobei die Kanüle (10) an einer Oberfläche, vorzugsweise der Außenoberfläche, mit einer kapillar wirksamen Oberflächenstruktur so ausgestattet ist, dass die Körperflüssigkeit aufgrund einer Kapillarwirkung der Oberflächenstruktur zur Messeinrichtung (23) transportiert wird.

2. Injektionsvorrichtung nach Anspruch 1 ,
**dadurch gekennzeichnet, dass**
die zumindest eine Messeinrichtung (23) zumindest ein eine Testchemie enthaltendes Testelement (6) aufweist.

3. Injektionsvorrichtung nach einem der vorhergehenden Ansprüche,
**dadurch gekennzeichnet, dass**
die zumindest eine Messeinrichtung (23) zumindest eine Komponente aus folgender Gruppe aufweist:
- zumindest einen Sensor zur Erfassung des Analytwertes,
- zumindest ein Leitungselement (7),
- eine elektrische und/oder optische Kontaktstelle (8) zum Datenaustausch.

4. Injektionsvorrichtung nach einem der vorhergehenden Ansprüche,
**dadurch gekennzeichnet, dass**
die Oberflächenstruktur als zumindest eine parallel und/oder zumindest teilweise quer zur Längsachse der Kanüle (10) verlaufende Kapillarrinne (18) ausgebildet ist.

5. Injektionsvorrichtung nach einem der vorhergehenden Ansprüche,
**dadurch gekennzeichnet, dass**
eine Oberfläche, vorzugsweise eine Außenoberfläche, der Kanüle (10) und/oder die Oberflächenstruktur zumindest teilweise mit einem hydrophilen Material beschichtet ist.

6. Injektionsvorrichtung nach einem der vorhergehenden Ansprüche,
**dadurch gekennzeichnet, dass**
die Oberfläche, vorzugsweise dieAußenoberfläche und/oder die Oberflächenstruktur zumindest teilweise mit einer Nanostruktur versehen ist.

7. Injektionsvorrichtung nach einem der vorhergehenden Ansprüche,
**dadurch gekennzeichnet, dass**
die Oberfläche, vorzugsweise die Außenoberfläche und/oder die Oberflächenstruktur zumindest teilweise eine plasmabehandelte Oberfläche aufweist.

8. Injektionsvorrichtung nach einem der vorhergehenden Ansprüche,
**dadurch gekennzeichnet, dass**
die Injektionsvorrichtung (1) mit einer Kanülenhalterung (2) ausgestattet ist, mit der die Injektionsvorrichtung (1) an eine Dosiervorrichtung (3) zur Dosierung der zu verabreichenden Flüssigkeit angebunden werden kann.

9. Injektionsvorrichtung nach Anspruch 8,
**dadurch gekennzeichnet, dass**
zumindest zwei der folgenden Komponenten integral als ein Einweg- und/oder Mehrwegartikel ausgebildet sind:
- die Kanüle (10),
- die Kanülenhalterung (2),
- das zumindest eine Testelement (6),
- der zumindest eine Sensor,
- die zumindest eine Kontaktstelle (8),
- das zumindest eine Leitungselement (7).

10. Injektionsvorrichtung nach Anspruch 8 oder 9,
**dadurch gekennzeichnet, dass**
der zumindest ein solches Testelement (6) aufweisende Einweg- und/oder Mehrwegartikel zwischen der Kanülenhalterung (2) und der Dosiervorrichtung (3) angeordnet ist.

11. Injektionsvorrichtung nach einem der Ansprüche 8 bis 10,
**dadurch gekennzeichnet, dass**
der zumindest ein solches Testelement (6) umfassende Einweg- und/oder Mehrwegartikel zwischen der Kanülenhalterung (2) und einer Kanülenspitze (16) angeordnet ist.

12. Injektionsvorrichtung nach einem der Ansprüche 8 bis 11,
**dadurch gekennzeichnet, dass**
die Kanülenhalterung (2) so ausgebildet ist, dass der zumindest ein solches Testelement (6) umfassende Einweg- und/oder Mehrwegartikel durch eine Öffnung (22) in der Kanülenhalterung (2) einschoben werden kann.

13. Injektionssystem mit einer eine Messeinrichtung (23) aufweisenden Injektionsvorrichtung (1), insbesondere nach einem der vorhergehenden Ansprüche, mit einer Dosiervorrichtung (3) zur Dosierung einer mittels der Injektionsvorrichtung (1) in einen Körper einzuspritzenden Flüssigkeit, mit einer Datenverarbeitungsvorrichtung zur Verarbeitung zumindest eines mittels der Messeinrichtung (23) aus einer Körperflüssigkeit bestimmten Analytwertes und mit einer zwischen der Dosiervorrichtung (3) und der Injektionsvorrichtung (1) angeordneten elektronischen und/oder optischen Kontaktstelle (8) zur Datenübermittlung.

14. Injektionssystem nach Anspruch 13,
**dadurch gekennzeichnet, dass**
die Datenverarbeitungsvorrichtung zumindest eine Komponente aus folgender Gruppe aufweist:
- eine Eingabeeinrichtung zur Eingabe von Informationen durch einen Benutzer,
- eine Ausgabeeinrichtung zur Informationsausgabe an den Benutzer,
- einen Datenspeicher zur Abspeicherung von Daten innerhalb des Injektionssystems,
- eine Schnittstelleneinrichtung zur Übertragung von Daten an periphere Geräte,
- einen elektrischen Energiespeicher.

15. Injektionssystem nach Anspruch 14,
**dadurch gekennzeichnet, dass**
die Ausgabeeinrichtung mit einer optischen und/oder akustischen und/oder haptischen Signalausgabe ausgestattet ist.
